# EUROPÄISCHE PATENTSCHRIFT

(11) **EP 3 957 291 B1**
(45) Veröffentlichungstag und Bekanntmachung des Hinweises auf die Patenterteilung: **07.06.2023**
(21) Anmeldenummer: 21187864.0
(22) Anmeldetag: 27.07.2021
(51) Int. Cl.: A61G 3/00, F16F 7/00, F16M 11/22, F16M 11/04, F16M 13/02

(54) **GERÄTEHALTEVORRICHTUNG, INSBESONDERE ZUR BEFESTIGUNG EINES MOBILEN MEDIZINISCHEN GERÄTES, UND MEDIZINISCHE VORRICHTUNG HIERMIT**
DEVICE HOLDER, IN PARTICULAR FOR MOUNTING A MOBILE MEDICAL DEVICE, AND MEDICAL DEVICE COMPRISING THE SAME
DISPOSITIF DE SUPPORT D'APPAREIL, EN PARTICULIER DESTINÉ À LA FIXATION D'UN APPAREIL MÉDICAL MOBILE ET DISPOSITIF MÉDICAL CORRESPONDANT

(30) Priorität: 28.07.2020 DE 102020119909
(43) Veröffentlichungstag der Anmeldung: 23.02.2022
(73) Patentinhaber: Schaub, Markus, 35580 Wetzlar (DE)
(72) Erfinder: Schaub, Markus, 35580 Wetzlar (DE)
(74) Vertreter: Patentanwälte Olbricht Buchhold Keulertz

(56) Entgegenhaltungen:
- WO-A1-2016/106727
- CN-U- 201 814 746
- CN-U- 210 510 956
- DE-A1-102008 039 651
- DE-A1-102012 025 066
- JP-U- H01 133 916
- US-A1- 2012 068 040

## Beschreibung

Die Erfindung betrifft eine Gerätehaltevorrichtung, insbesondere zur Befestigung eines mobilen medizinischen Gerätes aus der Gruppe Defibrillator, Sauerstoffgerät, Beatmungsgerät, Überwachungsmonitor, Infusionsgerät, Inhaliergerät, Elektrokardiograf, Transfusionspumpe, Lebenserhaltungssystem, Extrakorporales System und Herz-/Lungenmaschine (HLM) gemäß dem Oberbegriff von Anspruch 1 sowie eine medizinische Vorrichtung hiermit nach Anspruch 12.

Medizinische Geräte müssen bei der Patientenversorgung in Fahrzeugen gesichert werden, um eine zuverlässige Versorgung ohne Gefahrenquellen bereitstellen zu können. Hierzu dienen Gerätehaltevorrichtung, die beispielsweise dem Befestigen von Defibrillatoren, Beatmungsgeräten, Überwachungsmonitoren, Infusionsgeräten, Inhalatoren/Inhaliergeräten, Elektrokardiografen und Transfusionspumpen dienen.

So ist aus DE 10 2017 104 754 A1 eine Gerätehaltevorrichtung zur Befestigung von einem mobilen Sauerstoff- oder Beatmungsgerät für die mobile Intensivmedizin bekannt An einem, die an einem vertikal ausgerichteten Trägerelement festlegbar ist. Sie weist eine Mobileinheit und eine Befestigungseinheit auf. Die Mobileinheit verfügt über einen Aufnahmekörper, der einen Aufnahmeraum für ein Sauerstoff- oder Beatmungsgerät ausbildet. Hierzu gehört eine horizontal ausgerichtete Montageplatte, die auf der Oberseite Positionierungszapfen und seitliche Aufkantungen aufweist. Das so aufgenommene Gerät kann hierdurch nicht seitlich verrutschen. Die Mobileinheit hat ein erstes Einhängemittel und ein erstes Arretiermittel, die jeweils auf einer Rückseite des Aufnahmekörpers angeordnet sind, wobei das erste Arretiermittel höher angeordnet ist als das erste Einhängemittel. Die Befestigungseinheit weist eine Montageplatte auf, an der ein zweites Einhängemittel zum Einhängen des ersten Einhängemittels und ein zweites Arretiermittel zur arretierten Verbindung mit dem ersten Arretiermittel angeordnet sind.

Nachteilhaft an dieser Haltevorrichtung ist die hohe Spezialisierung auf mobile Sauerstoff- oder Beatmungsgeräte. Außerdem können Erschütterungen zu fehlerhaften Gerätefunktionen führen und das medizinische gerät auch dauerhaft beschädigen.

Aus US 2012 0068040 A1 ist eine Gerätehaltevorrichtung für Satellitenantennen bekannt, bei der eine Montageplatte über eine Dämpfungsvorrichtung mit einem Wandhalter verbunden ist. Die Montageplatte ist dabei im Wesentlichen eben ausgebildet, um Satellitenantennen unterschiedlicher Größe und Form tragen zu können.

Aufgabe der Erfindung ist es deshalb, eine Gerätehaltevorrichtung bereitzustellen, die eine hohe Variabilität hinsichtlich des eingesetzten Gerätes bietet und einen schadensfreien zuverlässigen Betrieb des Gerätes ermöglicht. Die technische Lösung soll möglichst komfortabel in der Handhabung und preiswert sein.

Hauptmerkmale der Erfindung sind im kennzeichnenden Teil von Anspruch 1 sowie Anspruch 12 angegeben. Ausgestaltungen sind Gegenstand der Ansprüche 2 bis 11.

Die Erfindung betrifft eine Gerätehaltevorrichtung, insbesondere zur Befestigung eines mobilen medizinischen Gerätes aus der Gruppe Defibrillator, Sauerstoffgerät, Beatmungsgerät, Überwachungsmonitor, Infusionsgerät, Inhaliergerät, Elektrokardiograf, Transfusionspumpe, Lebenserhaltungssystem, Extrakorporales System und Herz-/Lungenmaschine (HLM), mit einem Wandhalter, und mit einer Montageplatte, die im bestimmungsgemäßen Montagezustand zumindest im Wesentlichen oder exakt horizontal ausgerichtet ist und eine Oberseite, eine Unterseite und Seitenkanten aufweist. Die Montageplatte weist auf der Oberseite eine Befestigungseinrichtung zum Befestigen eines auf der Oberseite anzuordnenden medizinischen Gerätes auf. Außerdem weist die Gerätehaltevorrichtung eine Dämpfeinrichtung auf, über die die Montageplatte an dem Wandhalter festgelegt ist.

Vorteilhaft hieran ist, dass medizinische Geräte, die auf der Montageplatte angeordnet werden, gegenüber dem Wandhalter und damit gegenüber einem Befestigungsort wie einer Wand eines Krankenwagens, Helikopters, Flugzeuges oder Rettungsbootes, gedämpft gelagert sind. Hierdurch wird der sichere Betrieb des medizinischen Gerätes verbessert und Schäden werden vermieden. Die Dämpfeinrichtung sollte hierzu eine Dämpfungseigenschaft aufweisen, die im bestimmungsgemäßen Montagezustand vertikal wirkt. Weil die meisten medizinischen Geräte ohnehin für ein Aufstellen auf ebenem Untergrund vorbereitet sind, bietet sich die horizontal ausgerichtete Montageplatte an, um mit möglichst geringem Aufwand die Befestigung des medizinischen Gerätes sicherzustellen.

Gemäß einer speziellen Ausgestaltung ist die Dämpfeinrichtung zwischen einem Trägerelement des Wandhalters und der Montageplatte angeordnet, wobei das Trägerelement im bestimmungsgemäßen Montagezustand zumindest im Wesentlichen oder exakt horizontal ausgerichtet ist. Hierdurch gelingt eine steife Halterung und die Dämpfeinrichtung kann abseits von der biegebelasteten Zone zwischen Montageplatte und Wandhalter angeordnet sein. Die Ausrichtung ist relativ zum Befestigungsobjekt zu verstehen. Soweit sich das Befestigungsobjekt bewegt, beispielsweise ein Boot, Fluggerät oder Landfahrzeug ist, kann es zu geodätischen Lageänderung aufgrund der Fortbewegung und Fahrzeuglage kommen.

Optional kann das Trägerelement eine Trägerplatte aufweisen, die beabstandet unterhalb von der Montageplatte angeordnet ist. Dabei kann im Besonderen die Trägerplatte im bestimmungsgemäßen Montagezustand exakt oder zumindest im Wesentlichen parallel zur Montageplatte ausgerichtet sein. Hieraus resultiert eine stabile Unterkonstruktion unterhalb der Montageplatte. Auch bei einem Abstellen der Gerätehaltevorrichtung auf einem Untergrund wird das medizinische Gerät mittels der Dämpfeinrichtung vor Stößen und Vibrationen geschützt. An der Unterseite der Trägerplatte können optional Abstellfüße und/oder Fixierelemente für Airlineschienen bzw. Ladungssicherungsschienen angeordnet sein. Mittels Abstellfüßen würde ein sicherer Stand gewährleistet und die Trägerplatte nicht beschädigt, sowie beabstandet über etwaigem nassen Untergrund gehalten. Die optionalen Fixierelemente dienen einer zuverlässigen und schnellen Fixierung auf den Airlineschienen bzw. Ladungssicherungsschienen. Letztere haben vorzugsweise eine T-förmige Nut, in deren Nutöffnung in definierten Abständen Einfuhrlöcher, insbesondere kreisförmige Einführlöcher, ausgebildet sind. Auf Seiten der Gerätehaltevorrichtung haben die hierfür vorgesehenen optionalen Fixierelemente vorzugsweise Befestigungsköpfe, optional auch Aneinanderreihungen hiervon. Diese lassen sich in die Einführlöcher einstecken und dann in der Airlineschiene bzw. Ladungssicherungsschiene verschieben. Sobald die Befestigungsköpfe und Einführlöcher nicht mehr fluchten, ist eine Sicherung orthogonal zur Schiene hergestellt. Die Position entlang der Airlineschiene bzw. Ladungssicherungsschiene wird vorzugsweise mittels eines mechanisch lösbaren Rastelements gesichert, insbesondere mit einem Rundbolzen. Vorzugsweise ist ein solches Rastelement an der Trägerplatte gelagert, bevorzugt mit Federbelastung. Zur Betätigung bietet sich eine Variante mit Rasthebel an, mit der das Rastelement in eine Offenstellung bewegbar ist. Für den Rasthebel und das Rastelement kann beispielsweise ein Rastgehäuse auf der Oberseite der Trägerplatte angeordnet sein.

Bevorzugt ist die Montageplatte über die Dämpfeinrichtung schwimmend am Wandhalter gelagert. Entsprechend werden horizontal wirkende Stöße und Vibrationen gedämpft.

Im Speziellen kann die Dämpfeinrichtung zwischen ein und zwölf, vorzugsweise zwischen drei und 6, und besonders bevorzugt exakt vier Dämpfelemente auf der Unterseite der Montageplatte aufweisen. Ein Dämpfelement kann genügen, mit mehreren lassen sich aber insbesondere Taumelbewegungen und Rotationsschwingungen besser herunterdämpfen. Durch eine Verteilung der Dämpfelemente sinkt auch das Gesamtgewicht der Dämpfelemente in Summe. Als besonders vorteilhaft erweist es sich bei einer rechteckigen Grundform der Montageplatte exakt vier Dämpfelemente vorzusehen, nämlich je eines pro Ecke der rechteckigen Grundform. Es resultiert eine stabile Lagerung bei geringem Gewicht.

Es besteht die Möglichkeit, dass das oder die Dämpfelemente jeweils auf einer Trägeroberseite des Wandhalters angeordnet sind, wobei die Trägeroberseite im bestimmungsgemäßen Montagezustand zumindest im Wesentlichen oder exakt horizontal ausgerichtet ist. Damit steht die Montageplatte gewissermaßen auf den Dämpfelementen, die wiederum auf der Trägeroberseite sitzen.

Bei einer Ausführungsform ist das oder sind die Dämpfelemente jeweils durch einen Gummipuffer ausgebildet. Solche Gummipuffer sind preiswert und funktionieren über lange Zeit sicher und hygienisch unkritisch. Bevorzugt weist der Gummipuffer eine Gummischicht zwischen zwei Verbindungselementen auf. Die Gummischicht kann beispielsweise zwischen zwei Platten, bspw. Metallplatten, anvulkanisiert sein. Die Verbindungselemente können Schraubbolzen aufweisen, die insbesondere an den Metallplatten festgelegt sind. Optional können die Verbindungselemente über eine mechanische Sicherungskopplung miteinander verbunden sein, die bei einem Schaden oder Überlast der Gummischicht wirkt.

Optional kann die Montageplatte über eine mechanische Sicherungskopplung mit Bewegungsspiel an dem Wandhalter gesichert sein, wobei die Sicherungskopplung die Montageplatte an dem Wandhalter sichert, wenn die Dämpfeinrichtung überbelastet ist oder versagt. Entsprechend kann das medizinische Gerät nicht herabstürzen, wenn die Dämpfeinrichtung versagt. Außerdem wird die Dämpfeinrichtung vor Überlasten geschützt, wenn ein mechanischer Anschlag eine solche verhindert. Die mechanische Sicherungskopplung kann abseits der Dämpfeinrichtung ausgebildet sein oder aber sie ist Teil der Dämpfeinrichtung bzw. der Dämpfelemente.

Bei einer besonderen Ausführungsform weist der Wandhalter einen vertikalen Halteschenkel auf, auf dessen Vorderseite die Montageplatte angeordnet ist, und auf dessen gegenüberliegenden Rückseite Halteelemente zur Wandbefestigung angeordnet sind. Durch den vertikalen Halteschenkel wird eine gute Kraftableitung ohne große Biegemomente in eine Wand erzielt.

Bevorzugt weist der Wandhalter ein Haltegestell auf, das den vertikalen Halteschenkel ausbildet. Ein Gestell setzt sich aus Streben zusammen, wodurch ein geringes Gewicht bei hoher Steifigkeit erzielt wird. Die Trägerplatte kann am Haltegestell befestigt sein. Hierzu weist das Haltegestell vorzugsweise zwei beabstandet benachbart angeordnete L-Schenkel auf, und im bestimmungsgemäßen Montagezustand vertikal ausgerichtete Schenkel dieser L-Schenkel hiervon bilden den vertikalen Halteschenkel des Wandhalters aus. Im bestimmungsgemäßen Montagezustand horizontal ausgerichtete Schenkel dieser L-Schenkel können dann beabstandet unterhalb von der Montageplatte angeordnet sein.

Weiterhin können die Haltelemente Einhängehaken aufweisen. Hierdurch lässt sich der Wandhalter schnell an einer Befestigungsschiene oder dergleichen einhängen. Bevorzugt sind die Einhängehaken auf zwei Höhenlinien angeordnet, insbesondere um die Gerätehaltevorrichtung auf zwei parallel ausgerichteten Befestigungsschienen einzuhängen. Mithin resultiert eine stabile und gegen Kippen gesicherte Verbindung. Optional weisen die Halteelemente Rastelemente und/oder Schnellspanner auf, insbesondere um eine Schnellmontage an einer Befestigungsschiene, bspw. an einer Wand zu ermöglichen. Rastelemente sind besonders komfortabel, bieten jedoch nur begrenzt spielfreien Halt. Schnellspanner erlauben eine feste spielfreie Verbindung, lassen sich der Reihe nach lösen und erleichtern die Demontage.

Des Weiteren können die Halteelemente Schwenkhaken aufweisen, die zum Einhängen auf einem horizontal ausgerichteten Element um eine Achse schwenkbar gelagert sind, die im bestimmungsgemäßen Montagezustand exakt oder zumindest im Wesentlichen vertikal ausgerichtet ist. Mit Hilfe dieser kann die Gerätehaltevorrichtung auch an nicht dafür vorgesehenen Befestigungsorten eingehängt werden. Durch das Schwenken können sie zudem von der Gebrauchsstellung in eine Stauposition gebracht werden, in der sie nicht stören, insbesondere nicht mit einer Montagewand kollidieren. Zur Sicherung der Schwenkhaken in der Stauposition bietet sich eine Ausgestaltung an, bei der die Schwenkhaken in dieser Stauposition mit einem Federdruckstück gesichert sind. Um Lackbeschädigung an dem Teil zu verhindern, auf das die Schwenkhaken gehängt werden, bietet es sich an, die Schwenkhaken beschichtet auszuführen, so zum Beispiel mittels einer Gummibeschichtung. Ein typischer Anwendungsfall für das Einhängen mit den Schwenkhaken sind Fahrtragen ohne Befestigungsschiene.

Bei der erfindungsgemäßen Ausgestaltung weist die Befestigungseinrichtung auf der Oberseite der Montageplatte eine Aufnahmekontur zur formschlüssigen Aufnahme einer Geräteunterseite eines aufzunehmenden medizinischen Gerätes auf. Durch einen solchen Formschluss resultiert eine schnell herzustellende, stabile Befestigung in definierter Position. Die Geräteunterseite kann von einem Gehäuse oder einem Rohr(-rahmen-)gestell ausgebildet sein. Dabei kann die Aufnahmekontur eine gekrümmte Nut aufweisen. Eine solche verhindert Schiebebewegungen als auch Drehbewegungen, wenn ein formgleiches Negativ, insbesondere eine Rippe oder ein Rohr des medizinischen Gerätes hierin eingebracht ist.

Weiterhin kann die Befestigungseinrichtung auf der Oberseite der Montageplatte Sicherungselemente zur formschlüssigen Festlegung eines aufzunehmenden medizinischen Gerätes aufweisen. Optional sind die Sicherungselemente selbstsichernd ausgebildet, wozu sie vorzugsweise um eine vertikale Schwenkachse schwenkbar gelagert und einrastend gesichert sind. Hierdurch können die Sicherungselemente in eine Öffnung, über eine Sockelplatte oder über ein Rohr eines Rohrgestells des medizinischen Gerätes schwenken. Im Speziellen kann es sich bei den Sicherungselementen um schwenkbare Rasthebel handeln. Vorzugsweise weisen die Sicherungselemente Raststellungen auf, bspw. mit einem Federdruckstück (bspw. auch eine federnd gelagerte Kugel), die in Rastausnehmungen gedrückt wird. Insbesondere kann es hilfreich sein, die Sicherungselemente in einer Offenstellung zu sichern, damit das medizinische Gerät eingesetzt und einfach entnommen werden kann. Hilfreich ist es außerdem die Sicherungselemente in einer Geschlossenstellung zu sichern, damit das medizinische Gerät gut gesichert ist. Zum Schutz einer Beschichtung des medizinischen Gerätes bietet es sich an, die Sicherungselemente an den Kontaktflächen zu beschichten oder hier Gleitstücke vorzusehen, bspw. aus Polyamid.

In einer Ausführungsvariante weist die Montageplatte auf der Unterseite eine Stegstruktur auf, wobei in den Zwischenräumen der Stegstruktur Mulden ausgebildet sind. Hierdurch ist die Montageplatte steif und gleichzeitig sehr leicht.

Optional weist die Trägerplatte auf der Unterseite eine Stegstruktur auf, wobei in den Zwischenräumen der Stegstruktur Mulden ausgebildet sind. Hierdurch ist die Trägerplatte steif und gleichzeitig sehr leicht.

Aus Leichtbaugründen bietet sich eine Konstruktion an, gemäß der die Montageplatte wenigstens ein Plattenloch aufweist, wobei das oder die Plattenlöcher wenigstens 15 % Fläche der Oberseite der Montageplatte ausmachen.

Gleichermaßen bietet sich aus Leichtbaugründen eine Konstruktion an, gemäß der die Trägerplatte wenigstens ein Plattenloch aufweist, wobei die Plattenlöcher wenigstens 10 % der Fläche der Oberseite der Trägerplatte ausmachen.

In einer besonderen Ausführungsform weisen die Montageplatte und die Trägerplatte jeweils ein Plattenloch auf, die vertikal fluchtend angeordnet sind. Dies erlaubt es Blut-, Blutplasma oder sonstige Konserven von dem medizinischen Gerät oberhalb der Montageplatte durch die Montageplatte und die Trägerplatte nach unten hängen zu lassen. Hierzu sind die fluchtenden Plattenlöcher bevorzugt wenigstens 6 cm breit und wenigstens 8 cm lang. Dabei kann die Grundform dieser Löcher unter anderem rechteckig, rund oder oval sein.

Ergänzend kann die Gerätehaltevorrichtung einen Infusionsständer aufweisen, der im bestimmungsgemäßen Montagezustand exakt oder zumindest im Wesentlichen vertikal ausgerichtet ist. Hierdurch kann das medizinische Gerät und/oder der Patient direkt, mit einem Medikament oder Ähnlichem versorgt werden. Beim Transport genügt es die gesamte Gerätehaltevorrichtung zu transportieren, wodurch weniger Personal notwendig ist. Vorzugsweise weist der Infusionsständer im Bereich des oberen Endes einen Einhängehaken auf. Dieser dient dem Einhängen einer Flasche oder eines Beutels. Im bestimmungsgemäßen Montagezustand bildet der Infusionsständer vorzugsweise den höchsten Punkt der Gerätehaltevorrichtung aus. Bevorzugt ist der Infusionsständer am Wandhalter festgelegt. Hierdurch wirken die mastartigen Biegekräfte nicht auf die gedämpfte Montageplatte. Der Infusionsständer ist bevorzugt teleskopisch verlängerbar ausgebildet. Hierdurch kann er bei Nicht-Bedarf oder temporär beim Transport zusammengeschoben werden und stört weniger. Im Bereich der teleskopischen Verbindung zwischen zwei Ständerrohrstücken bietet es sich an, eine zwischen die Rohre ragende Manschette vorzusehen. Diese beschränkt den Reibkontakt auf den Bereich der Manschette und kann bei entsprechender Materialauswahl Geräuschentwicklungen vermeiden. Geeignet ist beispielsweise eine Manschette aus Kunststoff. Für eine sichere Arretierung bietet es sich an, zwischen zwei teleskopisch aneinander gelagerten Ständerrohrstücken ein Drucktasterelement bzw. ein Federmetallstück einzusetzen, das federnd in Rastlöcher eingreift.

Die Trägerplatte, die Montageplatte, das Haltegestell und der Infusionsständer können vorteilhafterweise aus einem Leichtbauwerkstoff ausgebildet sein, insbesondere aus Aluminium oder einer Polymerzusammensetzung oder aus einer Kombination hieraus.

Die Erfindung betrifft außerdem eine medizinische Vorrichtung mit einer Gerätehaltevorrichtung wie sie vor- und nachstehend beschrieben ist und mit einem mobilen medizinischen Gerät aus der Gruppe Defibrillator, Sauerstoffgerät, Beatmungsgerät, Überwachungsmonitor, Infusionsgerät, Inhaliergerät, Elektrokardiograf, Transfusionspumpe, Lebenserhaltungssystem, Extrakorporales System und Herz-/Lungenmaschine (HLM), wobei das mobile medizinische Gerät eine Gehäuseunterseite oder ein Rohrgestell aufweist, mit der bzw. dem das mobile medizinische Gerät auf der Oberseite der Montageplatte angeordnet und mit der Befestigungseinrichtung befestigt ist.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine perspektivische Ansicht einer Gerätehaltevorrichtung von vorne und schräg oben;
- Fig. 2: eine perspektivische Ansicht der Gerätehaltevorrichtung nach Fig. 1 von der Seite;
- Fig. 3: eine perspektivische Ansicht der Gerätehaltevorrichtung nach Fig. 1 von hinten und schräg oben; und
- Fig. 4: eine perspektivische Teilansicht der Gerätehaltevorrichtung nach Fig. 1 von unten.

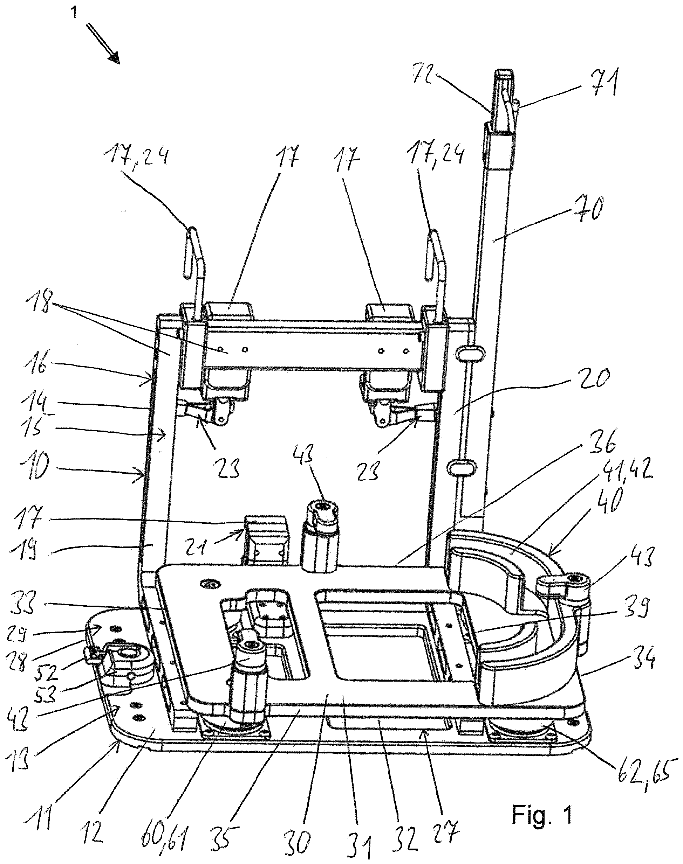

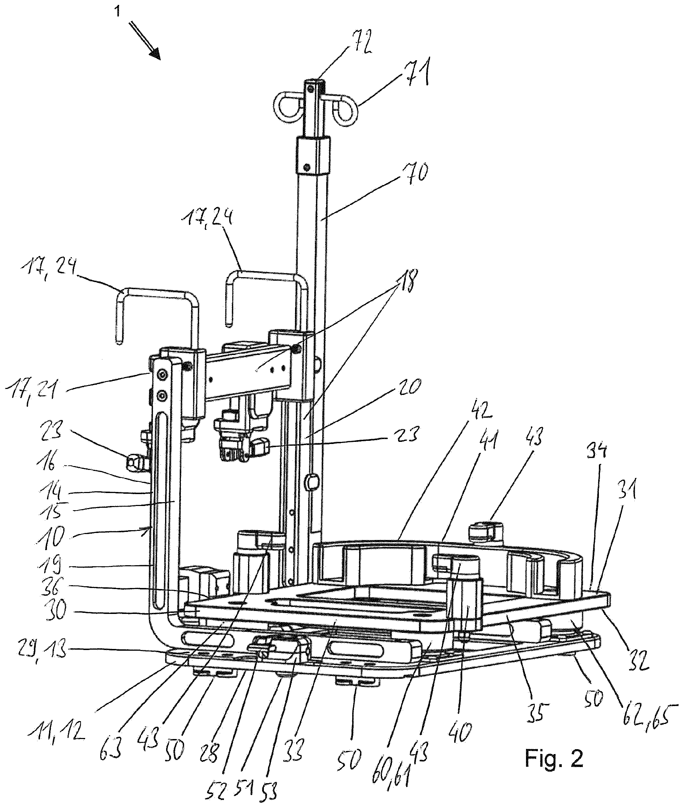

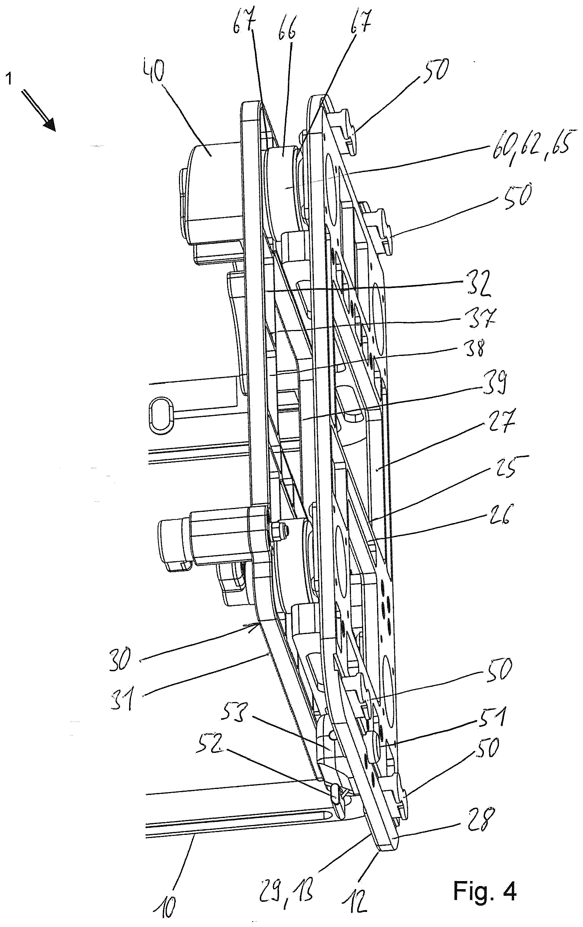

Die **Fig. 1****,** **2****,** **3** **und** **4** zeigen jeweils die gleiche Gerätehaltevorrichtung 1 aus unterschiedlichen Perspektiven, wobei Fig. 4 nur eine Teilansicht ist. Gleiche Bezugsziffern entsprechen damit gleichen Bauteilen und die Figuren werden gemeinsam beschrieben.

Die Gerätehaltevorrichtung 1 gemäß den Fig. **Fig. 1****,** **2****,** **3** **und** **4** dient der Befestigung eines mobilen medizinischen Gerätes aus der Gruppe Defibrillator, Sauerstoffgerät, Beatmungsgerät, Überwachungsmonitor, Infusionsgerät, Inhaliergerät, Elektrokardiograf, Transfusionspumpe, Lebenserhaltungssystem, Extrakorporales System und Herz-/Lungenmaschine (HLM). Sie weist einen Wandhalter 10 und eine Montageplatte 30 auf.

Die Montageplatte 30 ist im bestimmungsgemäßen Montagezustand zumindest im Wesentlichen oder exakt horizontal ausgerichtet und hat eine Oberseite 31, eine Unterseite 32 und Seitenkanten 33, 34, 35, 36. Auf der Oberseite 31 weist die Montageplatte 30 eine Befestigungseinrichtung 40 zum Befestigen eines auf der Oberseite 31 anzuordnenden medizinischen Gerätes auf.

Des Weiteren verfügt die Gerätehaltevorrichtung 1 über eine Dämpfeinrichtung 60 über die die Montageplatte 30 an dem Wandhalter 10 festgelegt ist. Die Dämpfeinrichtung 60 ist zwischen einem Trägerelement 11, insbesondere einer Trägerplatte 12, des Wandhalters 10 und der Montageplatte 30 angeordnet. Das Trägerelement 11, nämlich die Trägerplatte 12, ist im bestimmungsgemäßen Montagezustand zumindest im Wesentlichen oder exakt horizontal ausgerichtet, womit sie beabstandet unterhalb zur Montageplatte 30 angeordnet und zu dieser parallel ausgerichtet ist.

Wie man vor allem in Fig. 4 gut erkennen kann, sind an der Unterseite 28 der Trägerplatte 12 Fixierelemente 50 für Airlineschienen bzw. Ladungssicherungsschienen angeordnet. Die Fixierelemente 50 sind austauschbar ausgeführt und dienen einer zuverlässigen und schnellen Fixierung auf den Airlineschienen bzw. Ladungssicherungsschienen. Letztere haben vorzugsweise eine T-förmige Nut, in deren Nutöffnung in definierten Abständen Einfuhrlöcher, insbesondere kreisförmige Einführlöcher, ausgebildet sind. Auf Seiten der Gerätehaltevorrichtung haben die hierfür vorgesehenen Fixierelemente 50 Befestigungsköpfe, die teilweise aneinandergereiht ausgeführt sind, insbesondere jeweils paarweise. Diese Befestigungsköpfe lassen sich in die Einführlöcher einstecken und dann in der Airlineschiene bzw. Ladungssicherungsschiene verschieben. Sobald die Befestigungsköpfe und Einführlöcher nicht mehr fluchten, ist eine Sicherung orthogonal zur Schiene hergestellt. Die Position entlang der Airlineschiene bzw. Ladungssicherungsschiene wird mittels eines mechanisch lösbaren Rastelements 51 gesichert, insbesondere mit einem Rundbolzen. Dieses Rastelement ist an der Trägerplatte 12 gelagert, bevorzugt mit Federbelastung, und steht je nach Stellung unterschiedlich weit über die Unterseite 28 der Trägerplatte 12 hinaus. Zur Betätigung des Rastelements 51 ist ein Rasthebel 52 vorgesehen, mit der das Rastelement 51 in eine Offenstellung bewegbar ist. Der Rasthebel 52 und das Rastelement 51 sind in einem Rastgehäuse 53 gelagert, das auf der Oberseite 29 der Trägerplatte 12 angeordnet ist.

Außerdem erkennt man in Fig. 4, dass die Trägerplatte 12 auf der Unterseite 28 eine Stegstruktur 25 aufweist, sodass in den Zwischenräumen der Stegstruktur 25 Mulden 26 ausgebildet sind. Zur Gewichtsersparnis ist außerdem zwei Plattenlöcher 27 in der Trägerplatte 12 ausgebildet, die wenigstens 10 % Fläche der Oberseite 29 der Trägerplatte 12 ausmachen.

Der Wandhalter 10 bildet einen vertikalen Halteschenkel 14 aus, auf dessen Vorderseite 15 die Montageplatte 30 angeordnet ist, und auf dessen gegenüberliegender Rückseite 16 Halteelemente 17 zur Wandbefestigung angeordnet sind. Als Wand sollen alle vertikal ausgerichteten Bauelemente angesehen werden, die als Befestigungsuntergrund dienen können. Bevorzugt sind an dieser Wand Befestigungsschienen angebracht.

Teil des Wandhalters 10 ist ein Haltegestell 18, das den vertikalen Halteschenkel 14 ausbildet. Das Haltegestell 18 hat zwei beabstandet benachbart angeordnete L-Schenkel 19, 20, die über Quertraversen miteinander verbunden sind. Im bestimmungsgemäßen Montagezustand bildet ein vertikal ausgerichteter Schenkel dieser L-Schenkel 19, 20 den vertikalen Halteschenkel 14 des Wandhalters 10 aus bzw. sind Teil hiervon. Im bestimmungsgemäßen Montagezustand horizontal ausgerichtete Schenkel der L-Schenkel 19, 20 sind beabstandet unterhalb von der Montageplatte 30 jedoch auf der Trägerplatte 10 angeordnet.

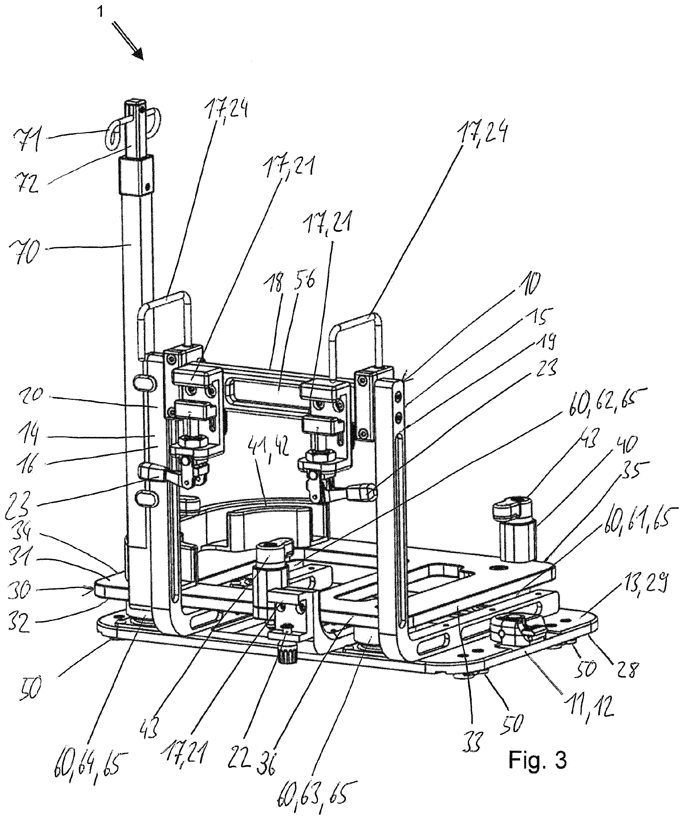

Die Haltelemente 17 auf der Rückseite 16 des vertikalen Halteschenkels 14 weisen Einhängehaken 21 auf (siehe insbesondere Fig. 3). Die drei Einhängehaken 21 sind auf zwei Höhenlinien angeordnet, um die Gerätehaltevorrichtung 1 auf zwei parallel ausgerichteten Befestigungsschienen einzuhängen. Das untere der Halteelemente 17 weist ein Rastelement 22 und die zwei oberen der Haltelemente 17 je einen Schnellspanner 23 auf.

Als zusätzliche Halteelemente 17 sind Schwenkhaken 24 vorgesehen, die zum Einhängen auf einem horizontal ausgerichteten Element um eine Achse schwenkbar gelagert sind, die im bestimmungsgemäßen Montagezustand exakt oder zumindest im Wesentlichen vertikal ausgerichtet ist. Wenn die Schwenkhaken 24 nicht gebraucht werden, werden sie so gedreht, dass sie aufeinander zeigen. In dieser Stauposition werden sie mittels je einem Federdruckstück 55 gehalten.

Für eine vereinfachte Handhabung ist in der oberen Quertraverse des Haltegestells 18 eine Mulde 56 ausgebildet. Diese dient vorallem der Gewichtsersparnis, kann jedoch vorliegend auch den Griff beim Tragen verbessern.

Die Trägerplatte 12 hängt nunmehr an der Unterseite der L-Schenkel 19, 20 befestigt am Haltegestell 18. Darüber ist die Montageplatte 30 über die Dämpfeinrichtung 60 schwimmend am Wandhalter 10 gelagert. Hierzu weist die Dämpfeinrichtung 60 exakt vier Dämpfelemente 61, 62, 63, 64 zwischen der Unterseite 31 der Montageplatte 30 und der Oberseite 29 der Trägerplatte 12 auf. Die Trägerplatte 12 hat eine rechteckige Grundform und je eines der Dämpfelemente 61, 62, 63, 64 sitzt im Bereich einer der Ecken der rechteckigen Grundform.

Wie insbesondere in Fig. 4 gekennzeichnet, sind die Dämpfelemente 61, 62, 63, 64 jeweils durch einen Gummipuffer 65 ausgebildet, der eine Gummizwischenschicht 66 zwischen zwei Verbindungselementen 67 aufweist. Je eines der zwei Verbindungselemente 67 ist an der Trägerplatte 12 festgelegt und eines an der Montageplatte 30.

Optional kann die Montageplatte 30 über eine mechanische Sicherungskopplung mit Bewegungsspiel an dem Wandhalter 10 gesichert sein, wobei die Sicherungskopplung die Montageplatte 30 an dem Wandhalter sichert, wenn die Dämpfeinrichtung 60 überbelastet ist oder versagt. Dabei kann die Sicherungskopplung auch im Kern der Gummizwischenschicht 66 ausgebildet sein, indem dort eine mechanische Verbindung mit Spiel zwischen den Verbindungselementen 67 ausgebildet ist.

Die Befestigungseinrichtung 40 auf der Oberseite 31 der Montageplatte 30 weist eine Aufnahmekontur 41 in Form einer gekrümmten Nut 42 zur formschlüssigen Aufnahme einer Geräteunterseite oder Rohrgestells eines aufzunehmenden medizinischen Gerätes auf. Zusätzlich verfügt die Befestigungseinrichtung 40 auf der Oberseite 31 der Montageplatte 30 über Sicherungselemente 43, insbesondere Rasthebel, zur formschlüssigen Festlegung eines aufzunehmenden medizinischen Gerätes. Die Sicherungselemente 43 sind selbstsichernd ausgebildet, wozu sie um eine vertikale Schwenkachse schwenkbar gelagert sind. Für ein Halten der Sicherungselemente 43 in einer geöffneten Position und einer geschlossenen Position sind Raststellungen vorgesehen. Hierzu ist zwischen dem Sicherungselement 43 und einer Unterkonstruktion jeweils ein Federdruckstück angeordnet, das in unterschiedlichen Drehwinkelstellungen des Sicherungselements 43 in gegenüberliegende Rastvertiefungen eingreift. j

Auch die Montageplatte 30 weist auf der Unterseite 32 eine Stegstruktur 37 auf, wobei in den Zwischenräumen der Stegstruktur 37 Mulden 38 ausgebildet sind. Zusätzlich sind in der Montageplatte 30 zwei Plattenlöcher 39 ausgebildet, die wenigstens 15 % Fläche der Oberseite 31 der Montageplatte 30 ausmachen. Dabei fluchtet eines der beiden Plattenlöcher 39 der Montageplatte 30 mit einem der beiden Plattenlöcher 27 der Trägerplatte 12, sodass ein Konservenbeutel durch die Montageplatte 30 und die Trägerplatte 12 hindurch nach unten hängen kann.

Parallel zum vertikalen Halteschenkel 14 erstreckt sich ein Infusionsständer 70, der mit dem vertikalen Halteschenkel 14 verbunden ist und im bestimmungsgemäßen Montagezustand exakt oder zumindest im Wesentlichen vertikal ausgerichtet ist. Im bestimmungsgemäßen Montagezustand bildet der Infusionsständer 70 den höchsten Punkt der Gerätehaltevorrichtung 1 aus.

Der Infusionsständer 70 ist telekopierbar mit einem ausziehbaren Teleskoprohr 72 ausgebildet. Am oberen Ende des Infusionsständer 70 ist ein Einhängehaken 71 ausgebildet. In dem Bereich, in dem das Teleskoprohr verschiebbar in einem Außenrohr 73 gelagert ist, sitzt eine Manschette 74 aus Kunststoff zwischen den beiden telekopierbaren Rohren 72, 73 und bildet so ein Gleitlager aus. Das Teleskoprohr 72 weist ein Federmetallstück 75 auf, das zum Eingreifen in Rastausnehmungslöcher des Außenrohres 73 ausgebildet ist. Das Außenrohr 73 ist mit dem Haltegestell 18 fest verbunden. Optional lässt sich ein Außenrohr des Infusionsständers 70 auch einteilig mit dem Haltegestell 18 ausbilden.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

**Bezugszeichenliste**

| | | | |
|---|---|---|---|
| 1 | Gerätehaltevorrichtung | 40 | Befestigungseinrichtung 41 |
| | | | Aufnahmekontur |
| 10 | Wandhalter | 42 | gekrümmte Nut |
| 11 | Trägerelement | 43 | Sicherungselement |
| 12 | Trägerplatte | | |
| 13 | Trägeroberseite | 50 | Sicherungselement |
| 14 | vertikaler Halteschenkel | 51 | Rastelement |
| 15 | Vorderseite (vertikaler | 52 | Rasthebel |
| | Halteschenkel) | 53 | Rastgehäuse |
| 16 | Rückseite (vertikaler Halteschenkel) | | |
| 17 | Halteelemente zur Wandbefestigung | 55 | Federdruckstück (Schwenkhaken) |
| 18 | Haltegestell | | |
| 19 | L-Schenkel | 56 | Mulde (Wandhalter) |
| 20 | L-Schenkel | | |
| 21 | Einhängehaken | 60 | Dämpfeinrichtung |
| 22 | Rastelement | 61 | Dämpfelement |
| 23 | Schnellspanner | 62 | Dämpfelement |
| 24 | Schwenkhaken | 63 | Dämpfelement |
| 25 | Stegstruktur | 64 | Dämpfelement |
| 26 | Mulde | 65 | Gummipuffer |
| 27 | Plattenloch (Trägerplatte) | 66 | Gummizwischenschicht |
| 28 | Unterseite (Trägerplatte) | 67 | Verbindungselemente |
| 29 | Oberseite (Trägerplatte) | | |
| | | 70 | Infusionsständer |
| 30 | Montageplatte | 71 | Einhängehaken |
| 31 | Oberseite | 72 | Teleskoprohr |
| 32 | Unterseite | 73 | Außenrohr |
| 33 | Seitenkante | 74 | Manschette |
| 34 | Seitenkante | 75 | Federmetallstück |
| 35 | Seitenkante | | |
| 36 | Seitenkante | | |
| 37 | Stegstruktur | | |
| 38 | Mulde | | |
| 39 | Plattenloch | | |

## Patentansprüche

1. **Gerätehaltevorrichtung** (1), insbesondere zur Befestigung eines mobilen medizinischen Gerätes aus der Gruppe Defibrillator, Sauerstoffgerät, Beatmungsgerät, Überwachungsmonitor, Infusionsgerät, Inhaliergerät, Elektrokardiograf, Transfusionspumpe, Lebenserhaltungssystem, Extrakorporales System und Herz-/Lungenmaschine (HLM),
- mit einem Wandhalter (10), und
- mit einer Montageplatte (30),
o die im bestimmungsgemäßen Montagezustand zumindest im Wesentlichen oder exakt horizontal ausgerichtet ist und eine Oberseite (31), eine Unterseite (32) und Seitenkanten (33, 34, 35, 36) aufweist,
o wobei die Montageplatte (30) auf der Oberseite (31) eine Befestigungseinrichtung (40) zum Befestigen eines auf der Oberseite (31) anzuordnenden medizinischen Gerätes aufweist,
∘ die Gerätehaltevorrichtung (1) eine Dämpfeinrichtung (60) aufweist, über die die Montageplatte (30) an dem Wandhalter (10) festgelegt ist
**dadurch gekennzeichnet, dass**
die Befestigungseinrichtung (40) auf der Oberseite (31) der Montageplatte (30) eine Aufnahmekontur (41) zur formschlüssigen Aufnahme einer Geräteunterseite eines aufzunehmenden medizinischen Gerätes aufweist.

2. Gerätehaltevorrichtung (1) gemäß Anspruch 1, **dadurch gekennzeichnet, dass** die Dämpfeinrichtung (60) zwischen einem Trägerelement (11) des Wandhalters (10) und der Montageplatte (30) angeordnet ist, wobei das Trägerelement (11) im bestimmungsgemäßen Montagezustand zumindest im Wesentlichen oder exakt horizontal ausgerichtet ist.

3. Gerätehaltevorrichtung (1) gemäß einem der Ansprüche 1 oder 2, **dadurch gekennzeichnet, dass** das Trägerelement (11) eine Trägerplatte (12) aufweist, die beabstandet unterhalb von der Montageplatte (30) angeordnet ist.

4. Gerätehaltevorrichtung (1) gemäß einem der vorhergehenden Ansprüche, **dadurch gekennzeichnet, dass** die Montageplatte (30) über die Dämpfeinrichtung (60) schwimmend am Wandhalter (10) gelagert ist.

5. Gerätehaltevorrichtung (1) gemäß einem der vorhergehenden Ansprüche, **dadurch gekennzeichnet, dass** die Dämpfeinrichtung (60) zwischen ein und zwölf Dämpfelemente (61, 62, 63, 64) auf der Unterseite (31) der Montageplatte (30) aufweist.

6. Gerätehaltevorrichtung (1) gemäß Anspruch 5, **dadurch gekennzeichnet, dass** das oder die Dämpfelemente (61, 62, 63, 64) jeweils durch einen Gummipuffer (65) ausgebildet sind.

7. Gerätehaltevorrichtung (1) gemäß einem der vorhergehenden Ansprüche, **dadurch gekennzeichnet, dass** die Montageplatte (30) über eine mechanische Sicherungskopplung mit Bewegungsspiel an dem Wandhalter (10) gesichert ist, wobei die Sicherungskopplung die Montageplatte (30) an dem Wandhalter (10) sichert, wenn die Dämpfeinrichtung (60) überbelastet ist oder versagt.

8. Gerätehaltevorrichtung (1) gemäß einem der vorhergehenden Ansprüche, **dadurch gekennzeichnet, dass** der Wandhalter (10) einen vertikalen Halteschenkel (14) aufweist, auf dessen Vorderseite (15) die Montageplatte (30) angeordnet ist, und auf dessen gegenüberliegender Rückseite (16) Halteelemente (17) zur Wandbefestigung angeordnet sind.

9. Gerätehaltevorrichtung (1) gemäß einem der vorhergehenden Ansprüche, **dadurch gekennzeichnet, dass** die Befestigungseinrichtung (40) auf der Oberseite (31) der Montageplatte (30) Sicherungselemente (43) zur formschlüssigen Festlegung eines aufzunehmenden medizinischen Gerätes aufweist.

10. Gerätehaltevorrichtung (1) gemäß einem der vorhergehenden Ansprüche, **dadurch gekennzeichnet, dass** die Montageplatte (30) auf der Unterseite (32) eine Stegstruktur (37) aufweist, wobei in den Zwischenräumen der Stegstruktur (37) Mulden (38) ausgebildet sind.

11. Gerätehaltevorrichtung (1) gemäß einem der vorhergehenden Ansprüche, **dadurch gekennzeichnet, dass** diese einen Infusionsständer (70) aufweist, der im bestimmungsgemäßen Montagezustand exakt oder zumindest im Wesentlichen vertikal ausgerichtet ist.

12. **Medizinische Vorrichtung** mit einer Gerätehaltevorrichtung (1) gemäß einem der vorhergehenden Ansprüche und mit einem mobilen medizinischen Gerät aus der Gruppe Defibrillator, Sauerstoffgerät, Beatmungsgerät, Überwachungsmonitor, Infusionsgerät, Inhaliergerät, Elektrokardiograf, Transfusionspumpe, Lebenserhaltungssystem, Extrakorporales System und Herz-/Lungenmaschine (HLM),
wobei das mobile medizinische Gerät eine Gehäuseunterseite oder Rohrgestell aufweist, mit der bzw. dem das mobile medizinische Gerät auf der Oberseite der Montageplatte angeordnet und mit der Befestigungseinrichtung befestigt ist.

## Claims

1. **Apparatus support device** (1), in particular for mounting a mobile medical device from the group defibrillator, oxygen device, respirator, surveillance monitor, infusion device, inhaler, electrocardiograph, transfusion pump, life support system, extracorporeal system and heart/lung machine (HLM),
- with a wall bracket (10), and
- with a mounting plate (30),
∘ which is aligned at least substantially or exactly horizontally in the intended assembly state and which has an upper side (31), a lower side (32) and side edges (33, 34, 35, 36),
∘ wherein, on the upper side (31), the mounting plate (30) has a fastening means (40) for fastening a medical device to be arranged on the upper side (31),
∘ the appliance holding device (1) comprises a damping device (60) via which the mounting plate (30) is fixed to the wall bracket (10)
**characterised in that,**
on the upper side (31) of the mounting plate (30), the fastening means (40) comprises a receiving contour (41) for form-fitting reception of a device underside of a medical device to be received.

2. Apparatus support device (1) according to claim 1, **characterised in that** the damping device (60) is arranged between a support element (11) of the wall bracket (10) and the mounting plate (30), the support element (11) being aligned at least substantially or exactly horizontally in the intended mounting state.

3. Apparatus support device (1) according to any one of claims 1 or 2, **characterised in that** the support element (11) comprises a support plate (12) spaced below the mounting plate (30).

4. Apparatus support device (1) according to one of the preceding claims, **characterised in that** the mounting plate (30) is floatingly mounted on the wall bracket (10) via the damping device (60).

5. Apparatus support device (1) according to any one of the preceding claims, **characterised in that** the damping device (60) comprises between one and twelve damping elements (61, 62, 63, 64) on the underside (31) of the mounting plate (30).

6. Apparatus support device (1) according to claim 5, **characterised in that** the damping element or elements (61, 62, 63, 64) are each formed by a rubber buffer (65).

7. Apparatus support device (1) according to any one of the preceding claims, **characterised in that** the mounting plate (30) is secured to the wall bracket (10) via a mechanical safety coupling with movement clearance, the safety coupling securing the mounting plate (30) to the wall bracket (10) when the damping device (60) is overloaded or fails.

8. Apparatus support device (1) according to one of the preceding claims, **characterised in that** the wall holder (10) comprises a vertical holding leg (14), the mounting plate (30) being arranged on a front side (15) of the vertical holding leg, and holding elements (17) for wall fastening being arranged on the opposite rear side (16) of the vertical holding leg.

9. Apparatus support device (1) according to one of the preceding claims, **characterised in that** the fastening means (40) comprises securing elements (43) on the upper side (31) of the mounting plate (30) for positively fixing a medical device to be accommodated.

10. Apparatus support device (1) according to one of the preceding claims, **characterised in that** the mounting plate (30) has a web structure (37) on the underside (32), wherein troughs (38) are formed in the intermediate spaces of the web structure (37).

11. Apparatus support device (1) according to one of the preceding claims, **characterised in that** it comprises an infusion stand (70) which is aligned exactly or at least substantially vertically in the intended assembly state.

12. **A medical device** comprising an apparatus support device (1) according to any one of the preceding claims and comprising a mobile medical device selected from the group consisting of a defibrillator, oxygen device, ventilator, surveillance monitor, infusion device, inhaler, electrocardiograph, transfusion pump, life support system, extracorporeal system and heart/lung machine (HLM),
wherein the mobile medical device comprises a housing base or tubular frame with which the mobile medical device is arranged on the upper side of the mounting plate and is fixed with the fastening means.

## Revendications

1. **Dispositif de support d'appareil** (1), en particulier pour la fixation d'un appareil médical mobile du groupe défibrillateur, appareil à oxygène, appareil respiratoire, moniteur de surveillance, appareil de perfusion, appareil d'inhalation, électrocardiographe, pompe de transfusion, système de maintien en vie, système extracorporel et machine coeur/poumons,
- avec un support mural (10), et
- avec une plaque de montage (30),
∘ qui, dans l'état de montage conforme, est orienté au moins essentiellement ou exactement à l'horizontale et présente une face supérieure (31), une face inférieure (32) et des bords latéraux (33, 34, 35, 36),
∘ dans lequel la plaque de montage (30) présente sur la face supérieure (31) un dispositif de fixation (40) pour fixer un appareil médical à disposer sur la face supérieure (31),
∘ le dispositif de support d'appareil (1) présente un dispositif d'amortissement (60) par lequel la plaque de montage (30) est fixée sur le support mural (10)
**caractérisé en ce que**
le dispositif de fixation (40) présente sur la face supérieure (31) de la plaque de montage (30) un contour de réception (41) pour la réception par complémentarité de forme d'une face inférieure d'un appareil médical à recevoir.

2. Dispositif de support d'appareil (1) selon la revendication 1, **caractérisé en ce que** le dispositif d'amortissement (60) est disposé entre un élément de support (11) du support mural (10) et la plaque de montage (30), l'élément de support (11) étant orienté au moins essentiellement ou exactement à l'horizontale dans l'état de montage conforme à l'utilisation.

3. Dispositif de support d'appareil (1) selon l'une des revendications 1 ou 2, **caractérisé en ce que** l'élément de support (11) comprend une plaque de support (12) qui est disposée à distance en dessous de la plaque de montage (30).

4. Dispositif de support d'appareil (1) selon l'une des revendications précédentes, **caractérisé en ce que** la plaque de montage (30) est montée de manière flottante sur le support mural (10) par l'intermédiaire du dispositif d'amortissement (60).

5. Dispositif de support d'appareil (1) selon l'une quelconque des revendications précédentes, **caractérisé en ce que** le dispositif d'amortissement (60) comprend entre un et douze éléments d'amortissement (61, 62, 63, 64) sur la face inférieure (31) de la plaque de montage (30).

6. Dispositif de support d'appareil (1) selon la revendication 5, **caractérisé en ce que** le ou les éléments d'amortissement (61, 62, 63, 64) sont chacun formés par un tampon en caoutchouc (65).

7. Dispositif de support d'appareil (1) selon l'une quelconque des revendications précédentes, **caractérisé en ce que** la plaque de montage (30) est fixée au support mural (10) par un couplage de sécurité mécanique avec un jeu de mouvement, le couplage de sécurité fixant la plaque de montage (30) au support mural (10) lorsque le dispositif d'amortissement (60) est surchargé ou tombe en panne.

8. Dispositif de support d'appareil (1) selon l'une des revendications précédentes, **caractérisé en ce que** le support mural (10) présente une branche de support verticale (14) sur la face avant (15) de laquelle est disposée la plaque de montage (30), et sur la face arrière opposée (16) de laquelle sont disposés des éléments de maintien (17) pour la fixation murale.

9. Dispositif de support d'appareil (1) selon l'une des revendications précédentes, **caractérisé en ce que** le dispositif de fixation (40) présente sur la face supérieure (31) de la plaque de montage (30) des éléments de sécurité (43) pour la fixation par complémentarité de forme d'un appareil médical à recevoir.

10. Dispositif de support d'appareil (1) selon l'une des revendications précédentes, **caractérisé en ce que** la plaque de montage (30) présente une structure d'entretoise (37) sur la face inférieure (32), des creux (38) étant formés dans les espaces intermédiaires de la structure d'entretoise (37).

11. Dispositif de support d'appareil (1) selon l'une des revendications précédentes, **caractérisé en ce qu'**il comporte une tige porte-sérum (70) qui est orientée exactement ou au moins sensiblement verticalement dans l'état de montage conforme à la destination.

12. **Dispositif médical** comprenant un dispositif de support d'appareil (1) selon l'une des revendications précédentes et comprenant un appareil médical mobile choisi parmi un défibrillateur, un appareil à oxygène, un respirateur, un moniteur de surveillance, un appareil de perfusion, un inhalateur, un électrocardiographe, une pompe à transfusion, un système de maintien en vie, un système extracorporel et une machine coeur-poumon,
dans lequel l'appareil médical mobile présente une face inférieure de boîtier ou un châssis tubulaire avec lequel l'appareil médical mobile est disposé sur la face supérieure de la plaque de montage et fixé avec le dispositif de fixation.
